(19) Europäisches Patentamt
European Patent Office
Office européen des brevets

(11) Publication number: **0 190 687**
**B1**

(12) **EUROPEAN PATENT SPECIFICATION**

(45) Date of publication of patent specification: **05.10.88**

(21) Application number: **86101313.4**

(22) Date of filing: **01.02.86**

(51) Int. Cl.$^4$: **C 07 C 101/34,** C 07 C 101/28, C 07 C 99/00

(54) **Process for preparing ethyl-alpha-(1-carboxyethyl)-amino-gamma-oxo-gamma-phenylbutyrate.**

(30) Priority: **04.02.85 JP 19483/85**
**12.08.85 JP 178396/85**

(43) Date of publication of application:
**13.08.86 Bulletin 86/33**

(45) Publication of the grant of the patent:
**05.10.88 Bulletin 88/40**

(84) Designated Contracting States:
**BE CH DE FR GB IT LI NL SE**

(56) References cited:
**DE-A-3 226 768**

**TETRAHEDRON LETTERS, vol. 25, no. 11, 1984, Oxford, New York, Paris, Frankfurt H. URBACH et al. "A favourable diasiereo-selective synthesis of N-(1-S-ethoxycarbonyl-3-phenylpropyl)-S-alanine" pages 1143-1146**

(73) Proprietor: **KANEGAFUCHI KAGAKU KOGYO KABUSHIKI KAISHA**
**2-4 Nakanoshima 3-chome**
**Kita-ku Osaka-shi Osaka-fu (JP)**

(72) Inventor: **Takahashi, Satomi**
**1-13-13, Shinwadai Tarumi-ku**
**Kobe-shi, Hyogo-ken (JP)**
Inventor: **Ueda, Yasuyoshi**
**2-63, Okihama-cho Takasago-cho**
**Takasago-shi, Hyogo-ken (JP)**
Inventor: **Yamada, Kazuhiko**
**248-3, Eigashima Okubo-cho**
**Akashi-shi, Hyogo-ken (JP)**
Inventor: **Yamada, Yukio**
**34-6, Minori Kakogawa-cho**
**Kakogawa-shi, Hyogo-ken (JP)**
Inventor: **Yamane, Takehiko**
**5-1-1121, Oakashi-cho 2-chome**
**Akashi-shi, Hyogo-ken (JP)**
Inventor: **Yanagita, Yoshifumi**
**2-63, Okihama-cho Takasago-cho**
**Takasago-shi, Hyogo-ken (JP)**
Inventor: **Shimada, Yoshio**
**321-2, Kawara Kakogawa-cho**
**Kagogawa-shi, Hyogo-ken (JP)**

**EP 0 190 687 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Watanabe, Kiyoshi**
**15-41, Matsugaoka 5-chome**
**Akashi-shi, Hyogo-ken (JP)**
Inventor: **Nomura, Michio**
**2-63, Okihama-cho Takasago-cho**
**Takasago-shi, Hyogo-ken (JP)**
Inventor: **Ohashi, Takehisa**
**9-14, Shinoharaobanoyama-cho 3-chome**
**Nada-ku**
**Kobe-shi, Hyogo-ken (JP)**

(74) Representative: **Türk, Gille, Hrabal**
**Bruckner Strasse 20**
**D-4000 Düsseldorf 13 (DE)**

## Description

The present invention relates to a process for preparing ethyl-α-(1-carboxyethyl)amino-γ-oxo-γ-phenylbutyrate, especially its optically active (αS,1S)-form having the formula (I):

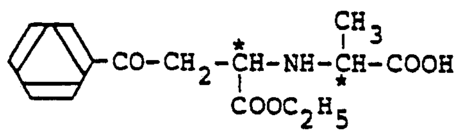

(I)

wherein an asterisk represents (S)-configuration of asymmetric carbon atom.

The compound having the formula (I) is a precursor of ethyl-(αS,1S)-α-(1-carboxyethyl)amino-γ-phenylbutyrate having the formula (III):

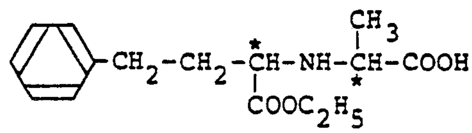

(III)

wherein an asterisk represents (S)-configuration of asymmetric carbon atom, which is a very useful intermediate compound for synthesis of the various amino acid derivatives having the general formula (II):

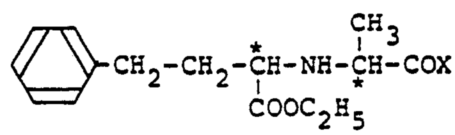

(II)

wherein X is

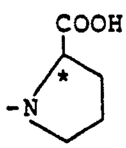 , 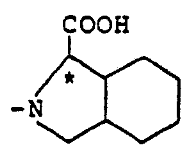 , 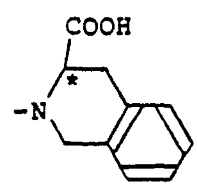

or the like and an asterisk represents (S)-configuration of asymmetric carbon atom, said amino acid derivatives having the general formula (II) being expected for use as antihypertensive agents due to their angiotensin converting enzyme (ACE) inhibitory activity.

Hitherto, it has been known that ethyl-α-(1-carboxyethyl)amino-γ-oxo-γ-phenylbutyrate is prepared by subjecting ethyl-β-benzoylacrylate (IV) and (S)-alanine benzyl ester (V) to the so-called Michael addition reaction in the presence of triethylamine and then conducting hydrogenolysis of the product to cleave benzyl group (Japanese Unexamined Patent Publication No. 103364/1983 and Tetrahedron Letters, Vol. 25 (11), 1143, (1984)). The reaction scheme is as follows:

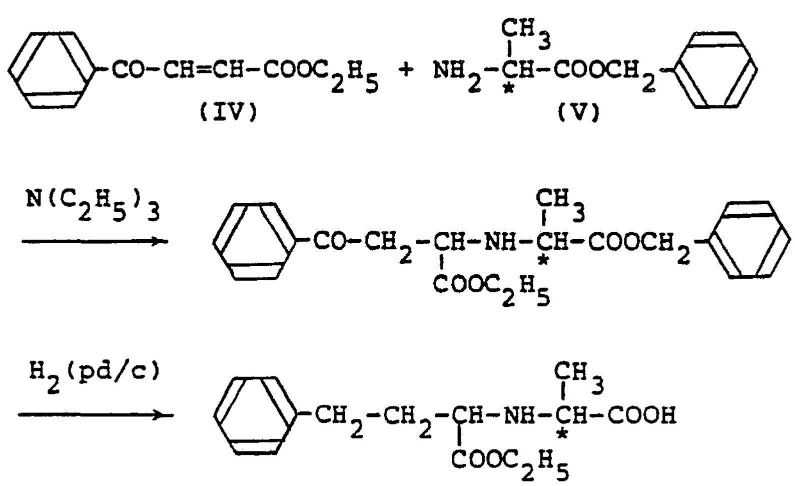


(αS,1S) --- (VI)

(αR,1S) --- (VII)

It is also confirmed that (αS,1S)-diastereoisomer (VI) is predominantly produced when (S)-alanine ester is employed and thus (αS,1S)-diastereoisomer (VI) can be obtained by crystallization or silica-gel column-chromatography.

However, the above method employing (S)-alanine ester requires a procedure to convert the amino group moiety of the ester, which is present in the form of a salt with an acid used in the esterification, into free amino group in addition to a procedure to esteify (S)-alanine. Further, it is required to employ such an ester that the ester moiety is selectively converted into carboxylic group by hydrogenoylsis without converting ethyl ester moiety of the product derived from ethyl-β-benzoylacrylate into carboxylic group. Thus the ester which can be employed in the reaction is limited to the ester such as benzyl ester or tert-butyl ester, which are prepared by relatively complicated procedure. Moreover, in order to carry out a selective ester degradation, the complicated procedure such as hydrogenolysis or trifluoroacetic acid treatment is required. Consequently, it is understood that the method is not suitable for an industrial product of (αS,1S)-ethyl-α-(1-carboxyethyl)-amino-γ-oxo-γ-phenylbutyrate in view point of an operatability and a cost for production.

As the result of the present inventors' continuous efforts to establish an economical, simple and efficient process for an industrial production of (αS,1S)-ethyl-α-(1-carboxyethyl)amino-γ-oxo-γ-phenylbutyrate, it was found that ethyl-α-(1-carboxyethyl)amino-γ-oxo-γ-phenylbutyrate can be obtained with an extremely high yield by reacting an alkali metal salt, an alkaline earth metal salt or a quarternary ammonium salt of alanine with ethyl-β-benzoylacrylate, that (αS,1S)-diasteroisomer of ethyl-α-(1-carboxyethyl)amino-γ-oxo-γ-phenylbutyrate is predominantly formed over (αR,1S)-diastereoisomer by conducting the reaction under particularly controlled reaction conditions employing a metal salt of (S)-alanine, that (αS,1S)-form is selectively crystallized only by adding an equivalent amount of an acid for neutralization and almost pure (αS,1S)-from is obtained by a simple procedure with an excellently high yield, and that ethyl-α-(1-carboxyethyl)amino-γ-phenylbutyrate can be prepared by catalytically reducing ethyl-α-(1-carboxyethyl)amino-γ-oxo-γ-phenylbutyrate.

According to the present invention, there can be provided a process for preparing ethyl-α-(1-carboxyethyl)amino-γ-oxo-γ-phenylbutyrate by reacting an alkali metal salt, an alkaline earth metal salt or a quarternary ammonium salt of alanine with ethyl-β-benzoylacrylate, wherein (αS,1S)-diastereoisomer is predominantly prepared over (αR,1S)-diastereoisomer by conducting the reaction under controlled conditions employing (S)-alanine, and a process for preparing ethyl-α-(1-carboxyethyl)amino-γ-phenylbutyrate by catalytically reducing ethyl-α-(1-carboxyethyl)amino-γ-oxo-γ-phenylbutyrate.

Starting trans-ethyl-β-benzoylacrylate can be easily prepared, for instance, by ethyl esterification of trans-β-benzoylacrylic acid, which is obtained by a known method such as Friedel-Crafts' acylation reaction of benzene and maleic anhydride or dehydration condensation of glyoxalic acid and acetophenone. Cis-ethyl-β-benzoylacrylate can be prepared by isomerizing the trans-form with irradiation of light.

The alkali metal salt, alkaline earth metal salt or quarternary ammonium salt of alanine can be prepared by a simple procedure such as stirring a mixture of alanine and a stoichiometrically necessary amount of alkali metal hydroxide, alkaline earth metal hydroxide, alkali metal carbonate, quarternary ammonium hydroxide or an ion exchange resin having quarternary ammonium hydroxide as an exchange group, in a solvent of water or alcohols at room temperature or with heating. If necessary, a metal salt of alanine can be isolated by distilling away the solvent under reduced pressure. Alternatively, a metal salt or quarternary ammonium salt of alanine can be prepared in situ in the reaction system by adding alkali metal hydroxide, alkaline earth metal hydroxide, alkali metal carbonate or quarternary ammonium hydroxide to a mixture of ethyl-β-benzoylacrylate and alanine.

Examples of quarternary ammonium employed in the reaction are, for instance, tetraalkyl ammonium such as tetramethyl ammonium, tetraethyl ammonium, tetrapropyl ammonium, tetrabutyl ammonium, tetrapentyl ammonium, tetrahexyl ammonium or tetraoctyl ammonium; or benzyltrimethyl ammonium, benzyltriethyl ammonium, cetyltrimethyl ammonium, decyltrimethyl ammonium, ethyltrimethyl ammonium, octyltrimethyl ammonium, phenyltrimethyl ammonium, trimethylstearyl ammonium, β-hydroxyethyltrimethyl ammonium, trioctylmethyl ammonium, tetradecyldimethylbenzyl ammonium and the like. Ammonium is also employed in the reaction. Further, an anion exchange, resin having a quarternary ammonium as an exchange group can also be employed though the reaction is carried out in a solid-liquid phase.

Michael addition reaction of ethyl-β-benzoylacrylate and an alkali metal salt, an alkaline earth metal salt or a quarternary ammonium salt of alanine can be carried out in a vast range of solvent such as, for instance, water, alcohols such as methanol, ethanol, propanol and butanol, chloroform, acetonitrile, n-hexane, dioxane, tetrahydrofuran or mixture thereof. The reaction is usually carried out in a solvent of alcohols.

Except for the reaction in a heterogeneous system, the addition reaction in a homogeneous system employing a solvent of alcohols proceeds very rapidly and is usually completed within several minutes to one hour at room temperature. Though the reaction may be carried out at a temperature ranging from −10 to 60°C, it is not preferable to conduct the reaction at a higher temperature since formed ethyl-α-(1-carboxyethyl)amino-γ-oxo-γ-phenylbutyrate is relatively unstable to the alkali in the reaction system. This unstability of the product in the reaction system is also observed after completion of the reaction as well as during the reaction and with the passage of time a decreased content of the product and a change in a ratio

of diastereoisomer are observed. However, such change of the product does not occur when the reaction system is acidified by adding not less than equivalent amount of an acid based on an employed alkali, especially mineral acid such as hydrochloric acid or sulfuric acid, to stabilize the product, which makes the subsequent operation much more easy.

Produced ethyl-α-(1-carboxyethyl)amino-γ-oxo-γ-phenylbutyrate can be easily isolated in a conventional manner by neutralizing the alkali with an acid and distilling away the solvent under reduced pressure, followed by crystallization. If necessary, after distilling away the solvent under reduced pressure, a residue is added with water and extracted with dichloromethane and the like at pH 3.5 to 5 to separate the product in a conventional manner. It is also possible to conduct the reduction of the product without isolation to give ethyl-α-(1-carboxyethyl)amino-γ-phenylbutyrate, which is then isolated.

Though the preferable reaction condition for selectively increasing a yield of (αS,1S)-diasteroisomer in the Michael addition reaction of ethyl-β-benzylacrylate and a metal salt or a quarternary ammonium salt of (S)-alanine varies depending on a combination of the agents employed in the reaction, it is mainly affected by the factors such as a kind of the salt of (S)-alanine, the reaction procedure and a concentration of the agents in the reaction. Lithium, sodium, potassium, magnesium, barium or calcium employed as an alkali metal or an alkaline earth metal. However, calcium is not suitable since it rather accelerate the production of (αR,1S)-di-asteroisomer. Though it is preferable to employ lithium salt or potassium salt of (S)-alanine when the reaction solvent is ethanol, different kind of (S)-alanine metal salt is employed depending on whether transform or cis-form of ethyl-β-benzoylacrylate is employed. That is, (αS,1S)-diasteroisomer is prepared with a higher yield by employing lithium salt of (S)-alanine when transethyl-α-benzoylacrylate is employed and by employing potassium salt of (S)-alanine when cis-ethyl-β-benzoylacrylate is employed.

In this way, optimum condition varies extremely depending on a kind of isomer of employed ethyl-β-benzoylacrylate and cannot sweepingly be determined. In case of trans-form of ethyl-β-benzoylacrylate, preferably lithium salt of (s)-alanine is slowly added for 5 minutes to 1 hour to an equivalent amount or an excess amount of ethyl-β-benzoylacrylate based on lithium salt of (S)-alanine and the reaction is preferably carried out in a relatively low concentration of 50 mM to 500 mM. On the other hand, in case of cis-form of ethyl-β-benzoylacrylate, potassium salt of (s)-alanine and ethyl-β-benzoylacrylate are preferably mixed together at a stretch in a higher concentration. When a quarternary ammonium salt of (S)-alanine is employed, an excess amount of ethyl-β-benzoylacrylate based on a quarternary ammonium salt of (S)-alanine is preferably employed in view point of a ratio of diastereoisomer and a yield. In any case, the addition reaction is completed within 5 minutes to 1 hour after completion of the addition of the reactants.

With comparison of trans-form and cis-form of ethyl-β-benzoylacrylate as a whole, there is a tendency that trans-form has a higher (αS,1S)/(αR,1S) ratio than that of cis-form. When the condition as mentioned above is employed, it is possible to give 4 to 5 of (αS,1S)/(αR,1S) ratio with trans-form and 2 to 3 with cis-form.

After completion of the addition reaction, an acid such as hydrochloric acid or sulfuric acid is immediately added to the reaction system to convert the formed metal salt or quarternary ammonium salt of ethyl-α-(1-carboxyethyl)amino-γ-oxo-γ-phenylbutyrate into ethyl-α-(1-carboxyethyl)amino-γ-oxo-γ-phenylbutyrate or hydrochloride or sulfate thereof, which is then isolated as a mixture of diasteroisomer. However, when the solvent of ethanol is used, optically almost pure (αS,1S)-ciastereoisomer is crystallized only by adding an equivalent amount of hydrochloric acid based on the alkali used and stirring the mixture while cooling the reaction mixture and thus only the desired compound can be isolated with an extremely high yield. By this procedure, complicated operation of the optical resolation indispensable to a usual synthesis reaction is not required and consequently (αS,1S)-ethyl-α-(1-carboxyethyl)amino-γ-oxo-γ-phenylbutyrate can be prepared very efficiently.

Alternatively, the reduction reaction can be carried out successively without isolation of ethyl-α-(1-carboxyethyl)amino-γ-oxo-γ-phenylbutyrate after adding not less than equivalent amount of sulfuric acid based on the alkali used in the addition reaction wherein ethanol is employed as a solvent. The catalytic reduction of ethyl-α-(1-carboxyethyl)amino-γ-oxo-γ-phenylbutyrate to ethyl-α-(1-carboxyethyl)amino-γ-phenylbutyrate proceeds with good yield in the presence of a small amount of acid such as sulfuric acid, hydrochloric acid or phosphoric acid in a polar protic solvent such as, for instance, alcohol, preferably ethanol, or carboxylic acid such as acetic acid. Examples of suitable catalyst are, for instance, Raney nickel, palladium, platinum and the like.

For example, when palladium carbon is employed as a catalyst, around 2 to around 70% of palladium carbon based on ethyl-α-(1-carboxyethyl)amino-γ-oxo-γ-phenylbutyrate is added to the reaction system and the catalytic reduction is conducted in a solvent of alcohol such as ethanol at 0° to 50°C, preferably 20° to 40°C, for several hours to 30 hours to almost quantitatively produce ethyl-α-(1-carboxyethyl)amino-γ-phenylbutyrate. Also the reaction time can be shortened by increasing the amount of the catalyst.

After completion of the reaction, the catalyst is separated, the acid is neutralized with an alkali such as, for instance, sodium hydroxide and the solvent is distilled away, followed by the optical resolution such as by recrystallization to give a highly pure crystal of (αR,1S)- or (αS,1S)-ethyl-α-(1-carboxyethyl)amino-γ-phenylbutyrate. When (αS,1S)-ethyl-α-(1-carboxyethyl)amino-γ-oxo-γ-phenylbutyrate is used as a starting material, (αS,1S)-ethyl-α-(1-carboxyethyl)-γ-phenylbutyrate can be obtained.

According to the process of the present invention and by controlling the conditions of the Michael addition reaction or work-up thereof as mentioned above, (αS,1S)-ethyl-α-(1-carboxyethyl)amino-γ-oxo-γ-phenylbutyrate having the formula (I) can be prepared in an extremely high yield starting from low-priced transethyl-β-benzoylacrylate or cis-ethyl-β-benzoylacrylate and a metal salt or quarternary ammonium salt of (S)-alanine. Further, according to the process of the present invention, (αS,1S)-ethyl-α-(1-carboxyethyl)-amino-γ-phenylbutyrate having the formula (III), which is an important intermediate compound for preparing angiotensin converting enzyme (ACE) inhibitory agents, can be prepared quite simply and efficiently.

The present invention is more particularly described by the following Examples and Reference Examples. However, it should be understood that the present invention is not limited, to the Examples and Reference Examples and various changes and modifications can be made without departing from the scope and spirit of the present invention.

In the high performance liquid chromatography (HPLC) analysis, sample solution was subjected to the analysis after sufficient acidification of the sample solution to be stabilized since ethyl-α-(1-carboxyethyl)-amino-γ-oxo-γ-phenylbutyrate is somewhat unstable under alkaline condition as previously described and (αS,1S)-diastereoisomer tends to be converted to (αR,1S)-diastereoisomer thermodynamically.

The HPLC analysis was conducted as follows:

Column: Finepak SIL $C_{18}$ (made by Japan Spectroscopic Co., Ltd.) (4.6 mm ID × 250 mm)
Mobile phase: 60 mM phosphate buffer (pH 2.5)/acetonitrile = 85/15 (V/V)
Flow rate: 1.5 ml/min.
Detection: 210 nm
Internal standard: 5-benzylhydantion

The instant HPLC analysis can separate and measure diastereoisomer such as (αS,1S) or (αR,1S)-form of ethyl-α-(1-carboxyethyl)amino-γ-oxo-γ-phenylbutyrate or ethyl-α-(1-carboxyethyl)amino-γ-phenylbutyrate and the analysis in the Examples was conducted by this method.

Example 1

A solution of 0.18 mmole of an alkali metal salt of (S)-alanine or 0.09 mmol of an alkaline earth metal salt of (S)-alanine dissolved in 0.5 ml of ethanol was quickly added to a solution of 37 mg of transethyl-β-benzoylacrylate (hereinafter referred to as "t-EBA") dissolved in 0.5 ml of ethanol at room temperature and the mixture was stirred for 5 minutes. Then the reaction was stopped by adding an acid to the reaction mixture and the HPLC analysis of the obtained product was conducted, which showed the production of ethyl-α-(1-carboxyethyl)amino-γ-oxo-γ-phenylbutyrate as shown in Table 1.

Table 1

| Alkali metal or alkaline earth metal | Amount of ethyl-α-(1-carboxyethyl)-amino-γ-oxo-γ-phenylbutyrate | |
|---|---|---|
| | (αS,1S)-form (mg) | (αR,1S)-form (mg) |
| Li | 80.8 | 17.3 |
| Na | 27.8 | 21.4 |
| K | 31.6 | 18.5 |
| Ca | 9.7 | 19.9 |

Example 2

A small test tube was charged with 30 mg of lithium salt of (S)-alanine and 2.5 ml of each solvent as shown in Table 2, to which 120 μl (135 mg) of t-EBA was added while stirring with a magnetic stirrer and the addition reaction was carried out. Then the reaction was stopped by adding an acid to the reaction mixture and the HPLC analysis of the obtained product was carried out, which showed the production of ethyl-α-(1-carboxyethyl)-amino-γ-oxo-γ-phenylbutyrate as shown in Table 2.

Table 2

| Solvent | Reaction time | Amount of ethyl-α-(1-carboxyethyl)amino-γ-oxo-γ-phenyl-butyrate | |
|---|---|---|---|
| | | (αS,1S)-form (mg) | (αR,1S)-form (mg) |
| Water-ethanol (1/1) | 10 minutes | 43.5 | 35.8 |
| Methanol | " | 38.9 | 28.3 |
| Ethanol | " | 46.5 | 35.2 |
| n-Propanol | 60 minutes | 43.5 | 37.1 |
| i-Propanol | " | 33.6 | 10.7 |
| n-Buthanol | " | 33.4 | 32.8 |
| i-Buthanol | " | 24.1 | 19.2 |
| Chloroform | 15 hours | 22.5 | 25.1 |
| Acetonitrile | " | 5.7 | 7.7 |
| n-Hexane | " | 8.8 | 6.5 |
| Dioxane | " | 8.9 | 10.1 |
| Tetrahydrofuran | " | 9.1 | 12.7 |
| Ethyl acetate/ethanol (1/1) | 30 minutes | 49.0 | 35.1 |

Example 3

A 100 ml three-neck round bottom flask was charged with 1.02 g of t-EBA, 223 mg of (S)-alanine and 30 ml of ethanol and the mixture was stirred with a magnetic stirrer at room temperature. By continuously adding 20 ml of ethanol solution containing 60 mg of lithium hydroxide to this suspension for 30 minutes the reaction mixture becoame gradually transparent and homogeneous. After stirring the mixture for 5 minutes, the reaction was stopped by adding 150 µl of sulfuric acid to the reaction mixture. The HPLC analysis of the obtained product was carried out, which showed the production of 676 mg of ethyl-α-(1-carboxyethyl)amino-γ-oxo-γ-phenylbutyrate ((αS,1S)-form/(αR,1S)-form) = 65/35).

Example 4

There was quickly added a solution of 46 mg of potassium salt of (S)-alanine dissolved in ethanol in an amount as shown in Table 3 to 73 mg of t-EBA at room temperature and the mixture was stirred for 5 minutes. The HPLC analysis of the obtained product was carried out as in Example 1.

The results are shown in Table 3.

Table 3

| Amount of ethanol (concentration) | Amount of ethyl-α-(1-carboxyethyl)-amino-γ-oxo-γ-phenylbutyrate | |
|---|---|---|
| | (αS,1S)-form (mg) | (αR,1S)-form (mg) |
| 0.3 ml (1.08 mol/ℓ) | 41.6 | 47.2 |
| 1.0 ml (0.36 mol/ℓ) | 52.2 | 38.7 |
| 3.0 ml (0.11 mol/ℓ) | 54.5 | 28.7 |

Example 5

There was added a solution of 34 mg of lithium salt of (S)-alanine dissolved in 1 ml of ethanol to 73 mg of t-EBA at room temperature at an addition rate as shown in Table 4 and the mixture was stirred for 3 minutes. The HPLC analysis of the obtained product was carried out as in Example 1.

The results are shown in Table 4.

Table 4

| Addition rate | Amount of ethyl-α-(1-carboxyethyl)-amino-γ-oxo-γ-phenylbutyrate | |
|---|---|---|
| | (αS,1S)-form (mg) | (αR,1S)-form (mg) |
| 1 mℓ/sec | 56.5 | 36.6 |
| 0.3 mℓ/min | 62.7 | 24.6 |

Example 6

There was added a solution of 46 mg of potassium salt of (S)-alanine dissolved in 1 ml of ethanol to a solution of 73 mg of t-EBA dissolved in ethanol of an amount as shown in Table 4 at room temperature for 3 minutes and the mixture was stirred for 3 minutes. The HPLC analysis of the obtained product was carried out as in Example 1.

The results are shown in Table 5.

Table 5

| Amount of ethanol (concentration) | Amount of ethyl-α-(1-carboxyethyl)-amino-γ-oxo-γ-phenylbutyrate | |
|---|---|---|
| | (αS,1S)-form (mg) | (αR,1S)-form (mg) |
| 0.5 ml (0.78 mol/ℓ) | 54.2 | 34.9 |
| 1.0 ml (0.36 mol/ℓ) | 59.2 | 31.4 |
| 2.0 ml (0.18 mol/ℓ) | 62.7 | 29.0 |

Example 7

The procedure of Example 1 was repeated except that cis-ethyl-β-benzoylacrylate (hereinafter referred to as "c-EBA") was employed in place of t-EBA.

The results are shown in Table 6.

Table 6

| Alkali metal or alkaline earth metal | Amount of ethyl-α-(1-carboxyethyl)-amino-γ-oxo-γ-phenylbutyrate | |
|---|---|---|
| | (αS,1S)-form (mg) | (αR,1S)-form (mg) |
| Li | 12.0 | 24.9 |
| Na | 22.9 | 19.3 |
| K | 29.3 | 15.5 |
| Ca | 11.1 | 19.6 |

Example 8

There was added a solution of 18 mg of lithium salt of (S)-alanine dissolved in 1.5 ml of ethanol to a solution of 114 mg of c-EBA dissolved in 3 ml of ethanol at room temperature for 5 minutes and the resultant was stirred for 3 minutes. The procedure of Example 1 was repeated and the HPLC analysis of the obtained product was carried out, which showed the production of 123 mg of ethyl-α-(1-carboxyethyl)-amino-γ-oxo-γ-phenylbutyrate ((αS1S)-form/(αR,1S)-form = 50/50).

Example 9

There was quickly added a solution of 118 mg of potassium salt of (S)-alanine dissolved in 0.5 ml of

ethanol to a solution of 190 mg of c-EBA dissolved in 0.5 ml of ethanol at room temperature and the miture was stirred for 3 minutes. The HPLC analysis of the obtained product was carried out as in Example 1, which showed the production of 245 mg of ethyl-α-(1-carboxyethyl)amino-γ-oxo-γ-phenylbutyrate ((αS,1S)-form/(αR,1S)-form = 69/31).

Example 10

There was added a solution of 0.603 g of lithium salt of (S)-alanine dissolved in 42.6 ml of ethanol to a solution of 2.59 g of t-EBA dissolved in 77 ml of ethanol at room temperature for 30 minutes. After completion of the addition, the mixture was stirred for 5 minutes and 0.529 ml of concentrated hydrochloric acid was added, which was then cooled with ice-water and the crystallization was carried out by adding 67.9 mg of (αS,1S)-form as seeds to the reaction mixture and stirring. After 4 hours, the precipitated crystals were filtered, washed with ethanol and dried to give 1.27 g of ethyl-α-(1-carboxyethyl)amino-γ-oxo-γ-phenylbutyrate ((αS,1S)-form/(αR,1S)-form = 95/5).

Melting point: 200° to 225°C (dec.)

$^1$H-nuclear magnetic resonance spectrum (DMSO-d6): 1.0 to 1.4 (t, 6H), 3.2 to 5.0 (m, 8H) and 7.3 to 8.1 (m, 5H)

Infrared absorption spectrum (cm$^{-1}$) (KBr disk): 3070, 1735, 1680, 1620 and 1580

$[\alpha]_D^{23}$ = +26.8 (c = 1.0, 1N HCl)

Example 11

There was quickly added a solution of 422 mg of potassium salt of (S)-alanine dissolved in 1.8 ml of ethanol to a solution of 680 mg of c-EBA dissolved in 1.8 ml of ethanol at room temperature and the mixture was stirred for 3 minutes. The reaction was stopped by adding 327 mg (3.3 mmol) of $H_2SO_4$ to the reaction mixture and the resultant was distributed between water and hexane. To the water layer was added 333 mg of triethylamine and extracted with dichloromethane three times. After dehydration of the dichloromethane layer with anhydrous magnesium sulfate, the solvent was removed by evaporation, followed by sufficient drying under reduced pressure to give 507 mg of ethyl-α-(1-carboxyethyl)amino-γ-oxo-γ-phenylbutyrate ((αS,1S)-form/(αR,1S)-form = 73/27).

Example 12

There was dissolved 0.4 g of ethyl-α-(1-carboxyethyl)amino-γ-oxo-γ-phenylbutyrate prepared in Example 10 into 8.0 ml of 1.6% (v/v) $H_2SO_4$-AcOH, to which 0.1 g of 10% Pd/C was added and the hydrogenation reaction was carried out at room temperature under atmospheric pressure. After completion of the reaction, the catalyst was filtered off with suction, to which 2.5 ml of 1N NaOH solution was added and the resultant was concentrated under reduced pressure. After the residue ws dissolved in water, a solution was adjusted to pH 3.0 and extracted with dichloromethane, the organic layer being washed with a saturated sodium chloride solution and concentrated under reduced pressure. The residue was crystallized from ethyl acetate to give 0.25 g of ethyl-α-(1-carboxyethyl)amino-γ-phenylbutyrate ((αS,1S)-form/(αR,1S)-form = 99/1).

Melting point: 149° to 149.5°C

$^1$H-Nuclear magnetic resonance spectrum ($CDCl_3$): 1.1 to 1.4 (t, 3H), 1,4 to 1,6 (d, 3H), 1.9 to 2,3 (m, 2H), 2.5 to 2.9 (m, 2H), 3.2 to 3.7 (m, 2H), 4.0 to 4.4 (q, 2H) and 6.9 to 7.4 (m, 5H)

Infrared absorption spectrum (cm$^{-1}$) (KBr disk): 3030, 2950, 1740 and 1600

$[\alpha]_D^{23}$ = +29.3 (c = 1.0, MeOH)

Example 13

There was dissolved 0.20 g of ethyl-α-(1-carboxyethyl)amino-γ-oxo-γ-phenylbutyrate prepared in Example 10 into 11.0 ml of 1% (v/v) $H_2SO_4$-EtOH, to which 0.05 g of 10% Pd/C was added and the hydrogenation reaction was carried out at room temperature under atmospheric pressure. After completion of the reaction, the catalyst was filtered off with suction and the ethanol solution was neutralized with sodium hydroxide, followed by distillation of the solvent under reduced pressure. The residue was dissolved in water and extracted with dichloromethane, the organic layer being concentrated under reduced pressure. The residue was crystallized from ethyl acetate to give 0.152 g of ethyl-α-(1-carboxyethyl)amino-γ-phenylbutyrate ((αS,1S)-form/(αR,1S)-form = 99/1).

Example 14

There was added a solution of 2.4 g of lithium salt of (S)-alanine dissolved in 160 ml of ethanol to 10.2 g of t-EBA dissolved in 300 ml of ethanol at room temperature for 30 minutes and the mixture was stirred for 5 minutes. The reaction was stopped by adding 4.4 g of sulfuric acid and the ethanol solution was concentrated under reduced pressure by distillation of ethanol and the residue was washed with n-hexane, to which 150 ml of acetic acid was added to dissolve the residue. To the resultant was added 1.65 g of 10% Pd/C and the hydrogenation reaction was carried out at room temperature under atmospheric pressure. After completion of the reaction, the catalyst was filtered off with suction. To the sulfuric acid-acetic acid solution was added 44.9 ml of 1N NaOH and the resultant was concentrated under reduced pressure. After the residue was dissolved in water, the solution was adjusted to pH 3 and extracted with 300 ml of

dichloromethane, the organic layer being washed with a saturated sodium chloride solution and concentrated under reduced pressure. The residue was crystallized from ethyl acetate to give 4.0 g of ethyl-α-(1-carboxyethyl)amino-γ-phenylbutyrate ((αS,1S)-form/(αR,1S)-form = 95/5).

Example 15

There was added a solution of 143.6 mg of lithium salt of (S)-alanine dissolved in 10 ml of ethanol to a solution of 616.0 mg of t-EBA dissolved in 18.2 ml of ethanol at room temperature for 30 minutes and the mixture was stirred for 5 minutes, each 5 ml of which was put into test tube, to which hydrochloric acid or sulfuric acid was added. The stability of the product with the passage of time was examined by the HPLC analysis.

The results are shown in Table 7.

Table 7

| Acid | Amount (mmole) | | Time (hour) 0 | 2 | 4 |
|---|---|---|---|---|---|
| HCl | 0.264 | (αS,1S)-form | 51.6 mg | 39.9 mg | 28.1 mg |
| | | (αR,1S)-form | 14.9 " | 13.0 " | 11.5 " |
| HCl | 0.528 | (αS,1S)-form | 51.6 " | 51.3 " | 51.5 " |
| | | (αR,1S)-form | 14.9 " | 14.8 " | 14.7 " |
| $H_2SO_4$ | 0.132 | (αS,1S)-form | 51.6 " | 32.4 " | 16.5 " |
| | | (αR,1S)-form | 14.9 " | 10.8 " | 10.1 " |
| $H_2SO_4$ | 0.264 | (αS,1S)-form | 51.9 " | 51.4 " | 51.5 " |
| | | (αR,1S)-form | 14.9 " | 14.8 " | 14.8 " |
| no addition | | (αS,1S)-form | 51.9 " | 37.9 " | 29.3 " |
| | | (αR,1S)-form | 14.9 " | 25.3 " | 30.5 " |

Example 16

There was added a solution of 0.332 g of lithium salt of (R)-alanine dissolved in 23 ml of ethanol to a solution of 1.42 g of t-EBA dissolved in 42 ml of ethanol at room temperature for 20 minutes. After completion of the reaction, the mixture was stirred for 3 minutes and 0.29 ml of concentrated hydrochloric acid was added, which was then cooled with ice-water. The crystallization was carried out by adding 20 mg of (αR,1R)-form as seeds. After 4 hours, the crystals were filtered off, washed with EtOH and dried to give 611 mg of ethyl-α-(1-carboxyethyl)amino-γ-oxo-γ-phenylbutyrate ((αR,1R)-form/(αS,1R)-form = 96/4)).

Melting point: 202° to 220°C (dec.)

$^1$H-Nuclear magnetic resonance spectrum (DMSO-$d_6$): 1.0 to 1.4 (t, 6H), 3.2 to 5.0 (m, 8H) and 7.3 to 8.1 (m, 5H)

Infrared absorption spectrum (cm$^{-1}$) (KBr disk): 3070, 1735, 1680, 1620 and 1580

$[D]_D^{23}$ = 26.7 (C = 1.0, 1N, HCl)

Reference Example 1

[Preparation of ethanol solution of a quarternary ammonium salt of (S)-alanine]

There was dissolved 25 mmole of various quarternary ammonium chloride into 12.5 ml of ethanol, to which 12.5 ml of ethanol solution containing 1.05 g (25 mmole) of sodium hydroxide was added and the mixture was sufficiently stirred. The formed NaCl precipitate was filtered to be removed. To the quarternary ammonium hydroxide solution 2.225 g (25 mmole) of the pulverized (S)-alanine was added and the mixture was stirred to form the salt, from which 50 ml of solution of the salt was prepared with ethanol (concentration: 0.5 m).

Reference Example 2

[Preparation of (S)-alanine carrier resin]

There was activated 100 ml of the most strongly basic anion exchange resin with 1N NaOH and sufficiently washed with water, to which 500 ml of an aqueous solution of 19.2 g of (S)-alanine was passed through, washed with water, and finally substituted with 150 ml of ethanol.

Example 17

A 50 ml three-neck flat bottom flask was charged with 1.75 g (8.6 mmole) of t-EBA and 25 ml of ethanol,

to which 8.6 ml (4.3 mmole) of an ethanol solution of a salt of (S)-alanine with quarternary ammonium as shown in Table 8 was continuously added at room temperature for 10 minutes while stirring with a magnetic stirrer and the mixture was further stirred for 6 minutes. There was added 0.5 ml of the obtained solution to 10 ml of 0.05 N HCl solution containing 10 mg of 5-benzylhydantoin of an internal standard to stop the reaction. The HPLC analysis of the product was carried out, which showed the production of ethyl-α-(1-carboxyethyl)amino-γ-phenylbutyrate as shown in Table 8.

Table 8

| Quarternary ammonium salt of (S)-alanine | Amount of ethyl-α-(1-carboxyethyl)amino-γ-oxo-γ-phenylbutyrate | |
|---|---|---|
| | (αS,1S)-form (mg) | (αR,1S)-form (mg) |
| Ammonium | 352.5 | 236.9 |
| Tetramethyl ammonium | 898.8 | 473.8 |
| Tetraethyl ammonium | 896.8 | 421.7 |
| Cetyltrimethyl ammonium | 837.3 | 415.0 |
| Trioctylmethyl ammonium | 824.2 | 429.1 |
| Tetra-n-butyl ammonium | 881.3 | 449.6 |
| Benzyltriethyl ammonium | 838.3 | 440.5 |
| Tetradecyldimethylbenzyl ammonium | 860.5 | 453.6 |
| Trimethylphenyl ammonium | 804.4 | 421.3 |
| Benzyldimethylphenyl ammonium | 608.5 | 320.5 |

Example 18

There was mixed 1.75 g of t-EBA and 383 g of (S)-alanine with 25 ml of ethanol. By adding continuously 8.6 ml of ethanol solution containing 907 mg of benzyltriethyl ammonium hydroxide to the above suspension at room temperature for 15 minutes while stirring, the reaction mixture became gradually transparent and homogeneous. After stirring for 6 minutes, the HPLC analysis of the product was carried out as in Example 17, which showed the production of 948.1 mg of ethyl-α-(1-carboxyethyl)amino-γ-oxo-γ-phenylbutyrate ((αS,1S)-form/(αR,1S)-form = 67.3/32.7).

Example 19

There was suspended 1.75 g of t-EBA in 25 ml of water-ethanol (1:1), to which 8.6 m of water-ethanol solution containing 4.3 mmole of ammonium salt of (S)-alanine was continuously added for 10 minutes. After stirring for 30 minutes, the HPLC analysis was carried out as in Example 17, which showed the production of 639 mg of ethyl-α-(1-carboxyethyl)amino-γ-oxo-γ-phenylbutyrate ((αS,1S)-form/(αR,1S)-form = 58.6/41.4)).

Example 20

There was dissolved 1.30 g of c-EBA in place of t-EBA in 20 ml of ethanol, to which 6.4 ml of the solution containing 3.2 mmole of tetramethyl ammonium salt of (S)-alanine as in Example 17 was continuously added at room temperature for 10 minutes. After stirring for 6 minutes, the HPLC analysis was carried out as in Example 17, which showed the production of 984.7 mg of ethyl-α-(1-carboxyethyl)amino-γ-oxo-γ-phenylbutyrate ((αS,1S)-form/(αR,1S)-form = 46.1/53.9).

Example 21

There was continuously added 8.6 ml of an ethanol solution containing 4.3 mmole of benzyltriethyl ammonium salt of (S)-alanine to a solution of 1.75 g of t-EBA dissolved in 25 ml of ethanol at room temperature for 10 minutes, which was then stirred for 6 minutes. The reaction solution was cooled with ice-water and adjusted to pH 4.5 with concentrated hydrochloric acid, to which 40 mg of (αS,1S)-form was added as seeds and the crystallization was carried out while stirring. After 4.5 hours, the crystals were filtered off, washed with ethanol and dried to give 0.698 g of ethyl-α-(1-carboxyethyl)amino-γ-oxo-γ-phenylbutyrate ((αS,1S)-form/(αR,1S)-form = 95.4/4.6).

Melting point: 200° to 225°C (dec.)

$^1$H Nuclear magnetic resonance spectrum (DMSO-d6): 1.0 to 1.4 (t, 6H), 3.2 to 5.0 (m, 8H) and 7.3 to 8.1 (m, 5H)

Infrared absorption spectrum (cm$^{-1}$) (KBr disk): 3070, 1735, 1680, 1620 and 1580

$[\alpha]_D^{23}$ = +26.8 (C = 1.0, 1N HCl)

Example 22

There was continuously added 9 ml of ethanol solution containing 4.5 mmole of tetramethyl ammonium salt of (S)-alanine to a solution of 1.83 g of t-EBA dissolved in 25 ml of ethanol at room temperature for 12 minutes while adjusting to the predetermined pH as shown in Table 9 with ethanol solution containing 2% acetic acid and the reaction was carried out with stirring. After stirring for 9 minutes, the HPLC analysis was carried out as in Example 17, which showed the production of ethyl-α-(1-carboxyethyl)amino-γ-oxo-γ-phenylbutyrate as shown in Table 19.

Table 9

| pH | Amount of ethyl-α-(1-carboxyethyl)-amino-γ-oxo-γ-phenylbutyrate | |
|---|---|---|
| | (αS,1S)-form (mg) | (αR,1S)-form (mg) |
| 11 | 833 | 437 |
| 12 | 867 | 437 |
| 13 | 636 | 311 |

Example 23

There was suspended (S)-alanine carrier resin of an ion exchange resin (estimated alanine content: 380 mg) in ethanol containing 1.75 g of EBAa to prepare 30 ml of suspension and the reaction was carried out at room temperature for the reaction time as shown in Table 10 while stirring slowly with magnetic stirrer. The suspension was filtered and the resin was washed with ethanol. The resultant resin was added to 10 ml of 1N-HCl and the mixture was stirred for 30 minutes, eluted twice and the obtained solution was added with ethanol to prepare 25 ml of a total amount. The HPLC analysis was carried out as in Example 17, which showed the production of ethyl-α-(1-carboxyethyl)amino-γ-oxo-γ-phenylbutyrate as shown in Table 10.

Table 10

| Ion exchange resin | Amount of resin (mℓ) | Reaction time (minutes) | Amount of ethyl-α-(1-carboxyethyl)amino-γ-oxo-γ-phenylbutyrate (mg) | |
|---|---|---|---|---|
| | | | (αS,1S)-form | (αR,1S)-form |
| AMBERLITE IRA-400 | 5 | 15 | 134.5 | 123.0 |
| | | 90 | 224.0 | 310.8 |
| IRA-400 IRA-410 | 4 | 15 | 41.5 | 31.5 |
| | | 90 | 70.0 | 80.3 |
| IRA-410 IRA-900 | 7.4 | 15 | 490.5 | 401.3 |
| | | 90 | 395.0 | 482.8 |
| AMBERLYST A-26 | 7.3 | 15 | 376.5 | 396.0 |
| | | 90 | 555.8 | 336.0 |

**Claims**

1. A process for preparing ethyl-α-(1-carboxyethyl)amino-γ-oxo-γ-phenylbutyrate, which comprises reacting an alkali metal salt, an alkaline earth metal salt or a quaternary ammonium salt of alanine with ethyl-β-benzoylacrylate.

2. The process of Claim 1, wherein an alkali metal or an alkaline earth metal or a quarternary ammonim hydroxide is added to the reaction mixture of alanine and ethyl-β-benzoylacrylate.

3. The process of Claim 1, wherein the quarternary ammonium, salt of alanine formed by an ion exchange resin having quarternary ammonium group as an exchange group is reacted.

4. The process of Claim 1, 2 or 3, wherein (S)-alanine is employed to prepare (αS,1S)- and (αR,1S)-ethyl-α-(1-carboxyethyl)amino-γ-oxo-γ-phenylbutyrate.

5. The process of Claim 1, 2 or 4, wherein lithium salt or potassium salt of alanine is reacted with trans-ethyl-β-benzoylacrylate to predominantly prepare (αS,1S)-ethyl-α-(1-carboxyethyl)amino-γ-oxo-γ-phenylbutyrate.

6. The process of Claim 1, 2 or 4, wherein potassium salt of (S)-alanine is reacted with cis-ethyl-β-benzoylacrylate to predominantly prepare (αS,1S)-ethyl-α-(1-carboxyethyl)amino-γ-oxo-γ-phenylbutyrate.

7. The process of Claim 1, 2, 3, 4, 5 or 6, wherein ethanol is employed as a reaction solvent.

8. The process of Claim 1, 2, 3, 4, 5, 6 or 7, wherein not less than equivalent amount of mineral acid is added to the reaction system after completion of the addition reaction in order to stabilize the formed products.

9. The process of Claim 1, 2, 3, 4, 5, 6, 7 or 8, wherein an acid is added to the reaction system after completion of the addition reaction to neutralize the alkali metal or quarternary ammonium salt and (αS,1S)-ethyl-α-(1-carboxyethyl)amino-γ-oxo-γ-phenylbutyrate is crystallized.

10. A process for preparing ethyl-α-(1-carboxyethyl)amino-γ--phenylbutyrate, which comprises reacting an alkali metal salt, an alkaline earth metal salt or quarternary ammonium salt of alanine with ethyl-β-benzoylacrylate to give ethyl-α-(1-carboxyethyl)amino-γ-oxo-γ-phenylbutyrate, and catalytically reducing the obtained ethyl-α-(1-carboxyethyl)amino-γ-oxo-γ-phenylbutyrate.

11. The process of Claim 10, wherein the catalytic reaction is carried out in ethanol containing mineral acid.

**Patentansprüche**

1. Verfahren zur Herstellung des Ethyl-α-(1-carboxyethyl)amino-γ-oxo-γ-phenylbutyrat, welches die Reaktion eines Alkalimetallsalzes, eines Erdalkalmetallsalzes oder eines quartären Ammoniumsalzes von Alanin mit Ethyl-β-benzoylacrylat umfaßt.

2. Verfahren nach Anspruch 1, in dem ein Alkalimetallhydroxid oder ein Erdalkalimetallhydroxid oder ein quartäres Ammoniumhydroxyd zu dem Reaktionsgemisch von Alanin und Ethyl-β-benzoylacrylat hinzugefügt wird.

3. Verfahren nach Anspruch 1, in dem das quartäre Ammoniumsalz von Alanin, das mittels eines Ionenaustauscherharzes mit quartärer Ammoniumgruppe als Austauschgruppe bebildet wurde, umgesetzt wird.

4. Verfahren nach Anspruch 1, 2 oder 3, in dem (S)-Alanin zur Herstellung von (αS,1S)- und (αR,1S)-Ethyl-α-(1-carboxyethyl)amino-γ-oxo-γ-phenylbutyrat eingesetzt wird.

5. Verfahren nach Anspruch 1, 2 oder 4, in dem das Lithiumsalz oder Kaliumsalz von Alanin mit trans-Ethyl-β-benzoylacrylat umgesetzt wird, zur Herstellung von vorwiegend (αS,1S)-Ethyl-α-(1-carboxyethyl)amino-γ-oxo-γ-phenylbutyrat.

6. Verfahren nach Anspruch 1, 2 oder 4, in dem das Kaliumsalz von (S)-Alanin mit cis-Ethyl-β-benzoylacrylat zur Herstellung von vorwiegend (αS,1S)-Ethyl-α-(1-carboxyethyl)amino-γ-oxo-γ-phenylbutyrat umgesetzt wird.

7. Verfahren nach Anspruch 1, 2, 3, 4, 5 oder 6, in dem Ethanol als ein Reaktionslösungsmittel verwendet wird.

8. Verfahren nach Anspruch 1, 2, 3, 4, 5, 6 oder 7, in dem nicht weniger als eine Äquivalentmenge einer Mineralsäure zu dem Reaktionssystem nach Vollendung der Additionsreaktion hinzugeführt wird, um die gebildeten Produkte zu stabilisieren.

9. Verfahren nach Anspruch 1, 2, 3, 4, 5, 6, 7 oder 8, in dem nach Vollendung der Additionsreaktion eine Säure zu dem Reaktionssystem hinzugefügt wird, um das Alkalimetall oder quartäre Ammoniumsalz zu neutralisieren, und in dem (αS,1S)-Ethyl-α-(1-carboxyethyl)amino-γ-oxo-γ-phenylbutyrat kristallisiert wird.

10. Verfahren zur Herstellung von Ethyl-α-(1-carboxyethyl)amino-γ-phenylbutyrat, welches umfaßt die Reaktion eines Alkalimetallsalzes, eines Erdalkalimetallsalzes oder eines quartären Ammoniumsalzes von Alanin mit Ethyl-β-benzoylacrylat, und Ethyl-α-(1-carboxyethyl)amino-γ-oxo-γ-phenylbutyrate zu geben, und die katalytische Reduktion des erhaltenen Ethyl-α-(1-carboxyethyl)amino-γ-oxo-γ-phenylbutyrat.

11. Verfahren nach Anspruch 10, in dem die katalytische Reaktion in Mineralsäure enthaltendem Ethanol durchgeführt wird.

**Revendications**

1. Un procédé pour préparer 1'α-(1-carboxyéthyl)amino-γ-oxo-γ-phénylbutyrate d'éthyle, qui comprend la réaction d'un sel de métal alcalin, d'un sel de métal alcalino-terreux ou d'un sel d'ammonium quaternaire d'alanine avec le β-benzoylacrylate d'éthyle.

2. Le procédé de la revendication 1, dans lequel on ajoute au mélange réactionnel d'alanine et de β-benzoylacrylate d'éthyle un hydroxyde de métal alcalin, de métal alcalino-terreux ou d'ammonium quaternaire.

3. Le procédé de la revendication 1, dans lequel le sel d'ammonium quaternaire d'alanine, formé par une résine échangeuse d'ions ayant un groupe ammonium quaternaire comme groupe échangeur, est mis à réagir.

4. Le procédé de la revendication 1, 2 ou 3, dans lequel ou utilise la (S)-alanine pour préparer 1'(αS,1S)- et 1'(αR,1S)-α-(1-carboxyéthyl)amino-γ-oxo-γ-phénylbutyrate d'éthyle.

5. Le procédé de la revendication 1, 2 ou 4, dans lequel on fait réagir le sel de lithium ou de potassium de l'alanine avec le trans-β-benzoylacrylate d'éthyle pour préparer de façon prédominante 1'(αS,1S)-α-(1-carboxyéthyl)amino-γ-oxo-γ-phénylbutyrate d'éthyle.

6. Le procédé de la revendication 1, 2 ou 4, dans lequel on fait réagir le sel de potassium de la (S)-alanine avec le cis-β-benzoylacrylate d'éthyl pour préparer de façon prédominate 1'(αS,1S)-α-((1-carboxy-éthyl)amino-γ-oxo-γ-phénylbutyrate d'éthyle.

7. Le procédé de la revendication 1, 2, 3, 4, 5 ou 6, dans lequel l'éthanol est utilisé comme solvant réactionnel.

8. Le procédé de la revendication 1, 2, 3, 4, 5, 6 ou 7; dans lequel on ajoute pas moins d'une quantité équivalente d'acide minéral au système réactionnel après achèvement de la réaction d'addition pour stabiliser les produits formés.

9. Le procédé de la revendication 1, 2, 3, 4, 5, 6, 7 ou 8, dand lequel ou ajoute un acide au système réactionnel après l'achèvement de la réaction d'addition pour neutraliser le sel de métal alcalin ou d'ammonium quaternaire et on cristallise 1'(αS,1S)-α-(1-carboxyéthyl)amino-γ-oxo-γ-phénylbutyrate d'éthyle.

10. Un procédé pour préparer 1'α-(1-carboxyéthyl)amino-γ-phénylbutyrate d'éthyle qui comprend la réaction d'un sel de métal alcalin, d'un sel de métal alcalino-terreux ou d'un sel d'ammonium quaternaire de l'alanine avec le β-benzoylacrylate d'éthyle pour former 1'α-(1-carboxyéthyl)amino-γ-oxo-γ-phénylbutyrate d'éthyle et la réduction catalytique de 1'α-(1-carboxyéthyl)amino-γ-oxo-γ-phénylbutyrate d'éthyle obtenu.

11. Le procédé de la revendication 10, dans leqquel la réaction catalytique est effectuée dans de l'éthanol contenant un acide minéral.